# EUROPEAN PATENT APPLICATION

(11) **EP 3 184 879 A1**
(43) Date of publication of application: **28.06.2017**
(21) Application number: 15851103.0
(22) Date of filing: 04.09.2015
(51) Int. Cl.: F21S 2/00, A61B 1/06

(54) **LIGHT SOURCE DEVICE**

(30) Priority: 17.10.2014 JP 2014212803
(71) Applicant: OLYMPUS CORPORATION, Hachioji-shi Tokyo 192-8507 (JP)
(72) Inventor: YABE, Yusuke, Tokyo 192-8507 (JP); KAMEE, Hiroyuki, Tokyo 192-8507 (JP); ISHIKAWA, Rihito, Tokyo 192-8507 (JP)
(74) Representative: Gunzelmann, Rainer
(86) International application number: PCT/JP2015/075217
(87) International publication number: WO 2016/059910

(57) **Abstract**

A light source device includes: a rotating body configured to be rotated with a rotating shaft as a center; a plurality of phosphors arranged on a plurality of circumferences of different radii with the rotating shaft as the center in the rotating body, and configured to be excited by being irradiated with light and generate fluorescence; and a light source portion configured to radiate excitation light for making one phosphor arranged on a predetermined circumference among the plurality of phosphors generate the fluorescence, and configured to radiate the excitation light to an area other than a straight line passing from the rotating shaft through an irradiation position at which the one phosphor is irradiated with the excitation light, in a phosphor different from the one phosphor among the plurality of phosphors.

## Description

### Technical Field

The present invention relates to a light source device including a laser diode as a light source and a rotating body provided with a phosphor that receives excitation light emitted from the light source and emits fluorescence.

### Background Art

In recent years, some light source devices have been configured using a light emitting element such as an LED or a laser diode instead of a lamp such as a discharge lamp or a filament lamp as a light source. The light emitting element is excellent in lighting responsiveness, lighting-out responsiveness and light adjustment responsiveness and is excellent in light emitting efficiency compared to the lamp.

Therefore, in the light source device with the light emitting element as the light source, irradiation of an object with light can be switched with excellent responsiveness by controlling lighting or lighting-out without disposing a shutter on an emission optical path like the light source device with the lamp as the light source.

In addition, by changing a driving current value or a driving voltage value to be inputted to the light emitting element, an emission light quantity can be adjusted with excellent responsiveness and accuracy. Therefore, need of a diaphragm for light adjustment provided on an optical path in the light source device with the lamp as the light source is eliminated.

Japanese Patent Application Laid-Open Publication No. 2011-145681 discloses a light emitting device capable of keeping light emission efficiency of a phosphor in an optimum state, a light source device configured by the light emitting device, and a projector including the light source device.

The light source device is configured including three light emitting devices that emit light of different wavelength regions respectively, and the light emitting device includes a light source, a rotating body where a phosphor layer that receives light radiated from the light source and emits predetermined wavelength region light is arranged, and a drive source that rotates the rotating body, or the like.

However, the light source device of the projector in Japanese Patent Application Laid-Open Publication No. 2011-145681 includes the three light emitting devices including the light source, the rotating body and the drive source or the like. Therefore, in the case of adopting a configuration of the light source device in Patent Literature 1 to a light source device for an endoscope, by providing three or more light emitting devices, a problem that the device is enlarged, and a problem that the device becomes expensive arise.

The present invention is implemented in consideration of above circumstances, and an object is to provide a light source device that prevents decline of wavelength conversion efficiency due to temperature rise of a phosphor while achieving miniaturization of the device and cost reduction.

### Disclosure of Invention

### Means for Solving the Problem

A light source device of one aspect of the present invention includes: a rotating body configured to be rotated with a rotating shaft as a center; a plurality of phosphors arranged on a plurality of circumferences of different radii with the rotating shaft as the center in the rotating body, and configured to be excited by being irradiated with light and generate fluorescence; and a light source portion configured to radiate excitation light for making one phosphor arranged on a predetermined circumference among the plurality of phosphors generate the fluorescence, and configured to radiate the excitation light to an area other than a straight line passing from the rotating shaft through an irradiation position at which the one phosphor is irradiated with the excitation light, in a phosphor different from the one phosphor among the plurality of phosphors.

A light source device of another aspect of the present invention includes: a rotating body configured to be rotated with a rotating shaft as a center and including a front surface and a rear surface; a control portion configured to control a drive portion that rotationally drives the rotating shaft in order to rotate the rotating body around the shaft; a front surface side wavelength conversion portion provided on a circumference with the rotating shaft as the center on the front surface of the rotating body, and configured to receive light and generate light of a wavelength different from a wavelength of the light; a rear surface side wavelength conversion portion provided on a circumference with the rotating shaft as the center on the rear surface of the rotating body, and configured to receive light and generate light of a wavelength different from a wavelength of the light; an irradiation portion configured to irradiate a predetermined position of the front surface side wavelength conversion portion provided on the front surface of the rotating body with light, and further irradiate an area different from the predetermined position of the rear surface side wavelength conversion portion provided on the rear surface of the rotating body with light; and a multiplexing portion configured to multiplex the light generated from the front surface side wavelength conversion portion and the light generated from the rear surface side wavelength conversion portion.

A light source device of yet another aspect of the present invention includes: a rotating body configured to be rotated with a rotating shaft as a center and including a front surface and a rear surface; a control portion configured to control a drive portion that rotationally drives the rotating shaft in order to rotate the rotating body around the shaft; a front surface side wavelength conversion portion provided on a circumference with the rotating shaft as the center on the front surface of the rotating body, and configured to receive light from an irradiation portion that radiates light and generate light of a wavelength different from a wavelength of the light; a rear surface side wavelength conversion portion provided on a circumference different from the front surface side wavelength conversion portion with the rotating shaft as the center on the rear surface of the rotating body, and configured to receive the light from the irradiation portion and generate light of a wavelength different from a wavelength of the light; and a multiplexing portion configured to multiplex the light generated from the front surface side wavelength conversion portion and the light generated from the rear surface side wavelength conversion portion.

### Brief Description of the Drawings

Fig. 1 is a diagram illustrating an endoscope system including a light source device relating to a first embodiment;
Fig. 2 is a schematic diagram illustrating a relation between one rotating body and four light source portions provided inside the light source device;
Fig. 3 is a diagram illustrating a configuration of the light source device;
Fig. 4 is a schematic diagram illustrating a relation between one rotating body and three light source portions provided inside the light source device;
Fig. 5 is a diagram illustrating an endoscope system including a light source device relating to a second embodiment;
Fig. 6 is a diagram of a front view from a front surface side of one rotating body provided in the light source device in Fig. 5, and is a diagram illustrating a wavelength conversion portion and an irradiation range or the like;
Fig. 7 is a diagram illustrating another configuration example of one rotating body provided in the light source device, relating to a modification of the light source device;
Fig. 8 is a diagram of a front view from the front surface side of the rotating body in Fig. 7, and is a diagram illustrating the wavelength conversion portion and the irradiation range or the like;
Fig. 9 is a diagram indicating another irradiation range of the rotating body in Fig. 8;
Fig. 10 is a diagram illustrating the light source device including two rotating bodies, relating to another configuration example of the light source device;
Fig. 11 is a diagram of a front view from the front surface side of the rotating body in Fig. 10, and is a diagram illustrating the wavelength conversion portion and the irradiation range or the like;
Fig. 12 is a diagram illustrating another configuration example of two rotating bodies provided in the light source device, relating to a different configuration example of the light source device; and
Fig. 13 is a diagram of a front view from the front surface side of the rotating bodies in Fig. 12, and is a diagram illustrating the wavelength conversion portion and the irradiation range or the like.

### Best Mode for Carrying Out the Invention

Hereinafter, embodiments of the present invention will be described with reference to the drawings.

Note that the individual drawings used in the following description are for schematic illustrations, a scale is made different for each component for dimensional relations and scales or the like of individual members in order to illustrate the individual components in such sizes that the components can be recognized on the drawings, and the present invention is not limited only to quantities of the components, shapes of the components, ratios of the sizes of the components, and relative positional relations of the individual components described in the drawings.

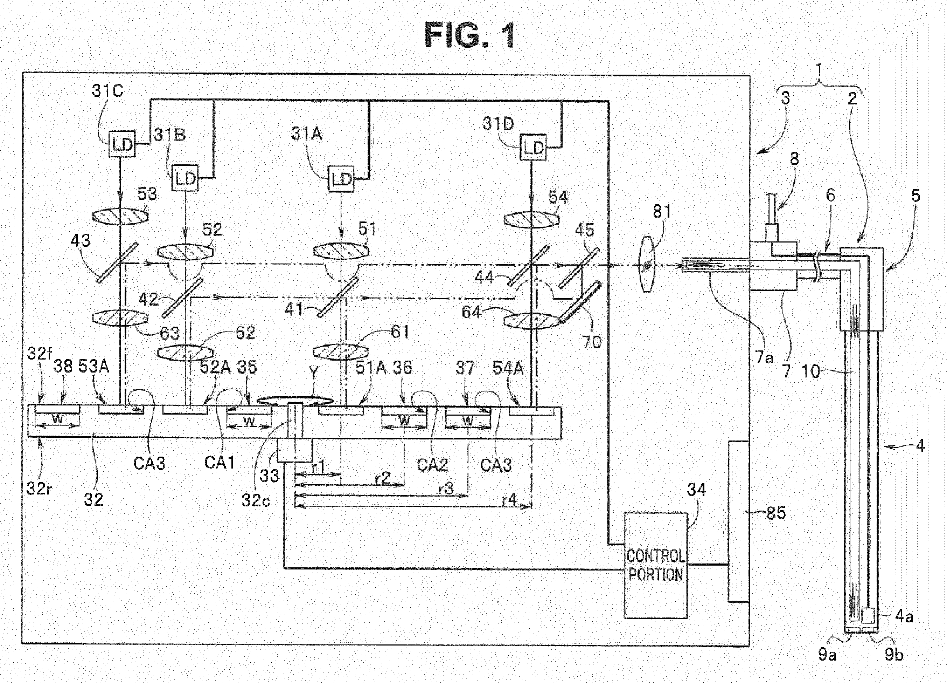

A light source device for an endoscope in a first embodiment will be described with reference to Fig. 1 to Fig. 3.

Fig. 1 is an endoscope system 1 configured mainly including an endoscope 2 and a light source device 3.

The endoscope 2 includes an image pickup apparatus 4a that picks up an image of an object inside a living body inside a distal end portion of an insertion portion 4, and image pickup signals photoelectrically converted in the image pickup apparatus 4a are outputted through a signal line 4b to a video processor not shown in the figure. The video processor converts the image pickup signals outputted from the endoscope 2 to video signals. Thereafter, the video signals are outputted from the video processor to a monitor (not shown in the figure), and an object image picked up in the image pickup apparatus 4a is displayed on a screen of the monitor.

A sign 5 denotes an operation portion and is provided on a proximal end side of the insertion portion 4. A sign 6 denotes a universal cord, and is extended from a side portion for example of the operation portion 5. On an end portion of the universal cord 6, a light source connector 7 is provided. From a side portion of the light source connector 7, an electric cable 8 is extended. An electric connector (not shown in the figure) provided on an end portion of the electric cable 8 is configured to be attached and detached to/from the video processor.

The light source connector 7 is configured to be attached and detached to/from the light source device 3.

A sign 9a denotes an illumination lens, a sign 9b denotes an image pickup lens, a sign 7a denotes a light guide pipe sleeve, and a sign 10 denotes a light guide fiber.

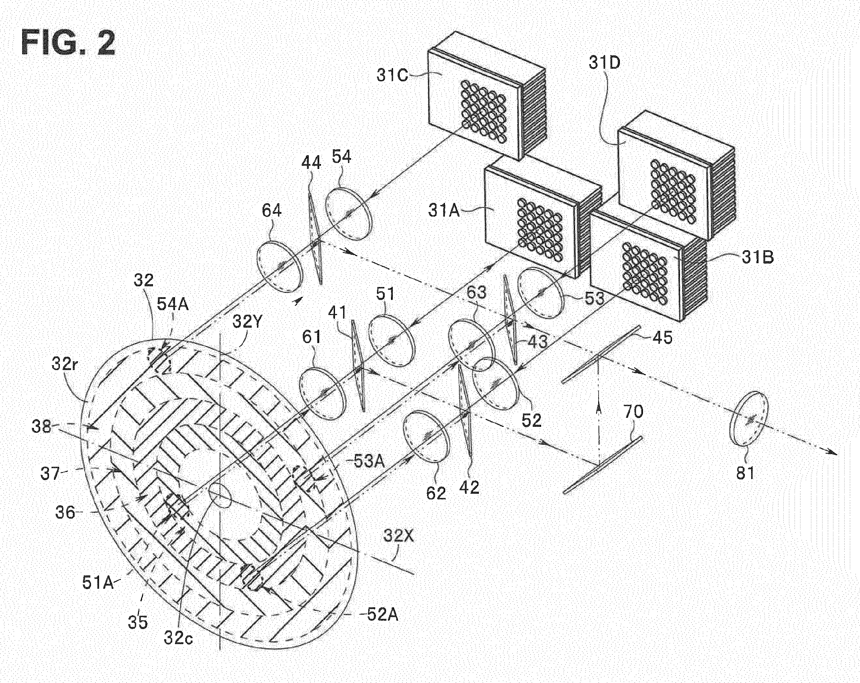

An inside of the light source device 3 is configured mainly including laser diodes (abbreviated as LD, hereinafter) 31 A, 31B, 31C and 31D as illustrated in Fig. 1 and Fig. 2 as light source portions, a rotating body 32, a motor 33, a control portion 34, a plurality of dichroic filters 41-46, a plurality of collimator lenses 51-54, and fluorescence pickup lenses 61-64.

The LDs 31 A, 31B, 31C and 31D are violet LDs 31 or ultraviolet LDs 31 for example that emit excitation light. In the present embodiment, four of a first LD 31 A, a second LD 31B, a third LD 31C and a fourth LD 31D are prepared as described above as the light source portions.

The rotating body 32 is a planar disk including a front surface 32f which is one flat surface and a rear surface 32r which is another flat surface that is an opposite surface of the front surface 32f. In the present embodiment, the four LDs 31A, 31B, 31C and 31D are provided on predetermined positions opposing the front surface 32f of the rotating body 32.

Note that the light source portion is not limited to the LD, and may be any light source as long as it is the excitation light, and an LED may be utilized.

On a center position of the rotating body 32, a rotating shaft 32c is integrally provided. The rotating shaft 32c is provided with the motor 33 as a drive portion that rotationally drives the rotating shaft 32c. By rotating the rotating shaft 32c by the motor 33, the rotating body 32 is rotated around the shaft.

On the front surface 32f of the rotating body 32, four kinds of annular wavelength conversion portions 35, 36, 37 and 38 are provided. A first wavelength conversion portion 35, a second wavelength conversion portion 36, a third wavelength conversion portion 37, and a fourth wavelength conversion portion 37 are configured including phosphors.

The first wavelength conversion portion 35, the second wavelength conversion portion 36, the third wavelength conversion portion 37, and the fourth wavelength conversion portion 37 receive light from the LDs 31A, 31B, 31C and 31D, and emit fluorescence of respectively different wavelengths that realize color reproducibility suitable for endoscope observation.

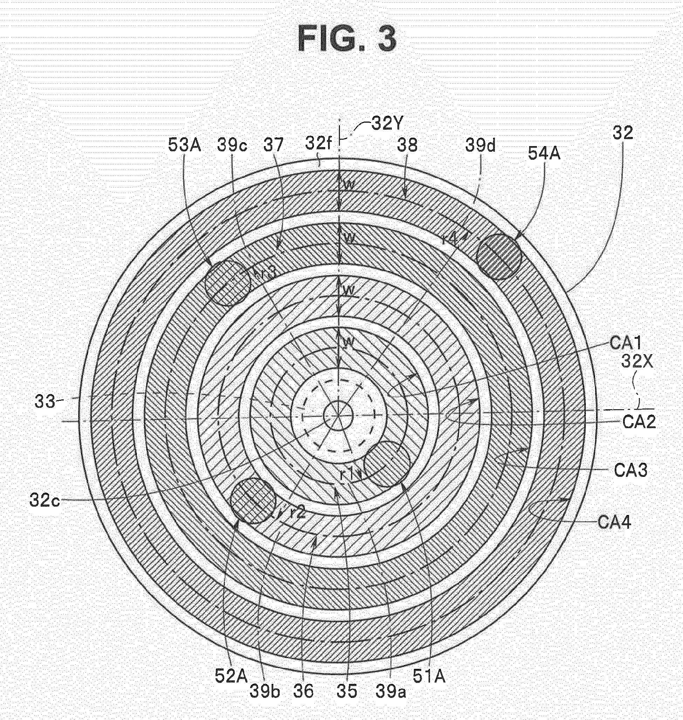

As illustrated in Fig. 3, the first wavelength conversion portion 35 is configured including a first phosphor in a first annular area CA1 formed along a first circle 39a of a radius r1 separated from a center of the rotating shaft 32c by a first distance r1. A width of the first annular area CA1 is w for example. The first wavelength conversion portion 35 is an annular portion of the width w with a circumference of the first circle 39a as a center line.

The second wavelength conversion portion 36 is configured including a second phosphor in a second annular area CA2 formed having the width w with a second circle 39b of a radius r2 larger than the radius r1 separated from the center of the rotating shaft 32c by a second distance r2 as the center line.

Here, the radius r2 is set such that the second wavelength conversion portion 36 is not arranged overlapping with the first wavelength conversion portion 35.

The third wavelength conversion portion 37 is configured including a third phosphor in a third annular area CA3 formed having the width w along a third circle 39c of a radius r3 larger than the radius r2 separated from the center of the rotating shaft 32c by a third distance r3.

Here, the radius r3 is set such that the third wavelength conversion portion 37 is not arranged overlapping with the second wavelength conversion portion 36.

The fourth wavelength conversion portion 38 is configured including a fourth phosphor in a fourth annular area CA4 formed having the width w along a fourth circle 39d of a radius r4 larger than the radius r3 separated from the center of the rotating shaft 32c by a fourth distance r4.

Here, the radius r4 is set such that the fourth wavelength conversion portion 38 is not arranged overlapping with the third wavelength conversion portion 37.

That is, the first wavelength conversion portion 35, the second wavelength conversion portion 36, the third wavelength conversion portion 37, and the fourth wavelength conversion portion 38 are annular portions formed having the width w with the circumferences of the individual circles 39a, 39b, 39c and 39d as the center lines. The annular areas CA1, CA2, CA3 and CA4 which are the annular portions are arranged without overlapping in order from the center of the rotating shaft 32c.

The first phosphor receives the excitation light emitted from the first LD 31 A, and generates blue fluorescence for example of a wavelength different from the wavelength of the excitation light.

The second phosphor receives the excitation light emitted from the second LD 31B, and generates red fluorescence for example of a wavelength different from the wavelengths of the excitation light and the blue fluorescence.

The third phosphor receives the excitation light emitted from the third LD 31C, and generates green fluorescence for example of a wavelength different from the wavelengths of the excitation light, the blue fluorescence and the red fluorescence.

The fourth phosphor receives the excitation light emitted from the fourth LD 31D, and generates umber fluorescence for example of a wavelength different from the wavelengths of the excitation light, the blue fluorescence, the red fluorescence and the green fluorescence.

The control portion 34 supplies motor drive signals to the motor 33, and controls a rotation speed of the motor 33. In addition, the control portion 34 supplies a driving current to the individual LDs 31 A, 31B, 31C and 31D respectively and adjusts emission light quantities from the individual LDs 31A, 31B, 31C and 31D.

Then, in order to prevent overlapping of irradiation ranges (also referred to as irradiation positions) in the phosphors of the first LD 31A, the second LD 31B, the third LD 31C, and the fourth LD 31D which are the light source portions, excitation light irradiation positions of the individual LDs 31A, 31B, 31C and 31D in an area other than a straight line passing through the individual irradiation positions from the rotating shaft 32c.

Then, in the present embodiment, setting is specifically performed as follows and each fluorescence is obtained from each phosphor.

The first LD 31A which is a first irradiation portion is provided facing the first wavelength conversion portion 35 positioned inside a fourth quadrant quadrisected by an X axis 32X of the rotating body 32 and a Y axis 32Y orthogonal to the X axis 32X as illustrated in Fig. 2 and Fig. 3. The excitation light indicated by a solid line, which is emitted from the first LD 31A, is converged by a first collimator lens 51 and radiated toward a first irradiation range (see a sign 51A indicated by a broken line in Fig. 2 and a solid line in Fig. 3), and the blue fluorescence is generated from the first irradiation range 51A.

The second LD 31B which is a second irradiation portion is provided facing the second wavelength conversion portion 36 positioned inside a third quadrant. The excitation light indicated by a solid line, which is emitted from the second LD 31B, is converged by a second collimator lens 52 and radiated toward a second irradiation range (see a sign 52A indicated by a broken line in Fig. 2 and a solid line in Fig. 3), and the red fluorescence is generated from the second irradiation range 52A.

The third LD 31C which is a third irradiation portion is provided facing the third wavelength conversion portion 37 positioned inside a second quadrant. The excitation light indicated by a solid line, which is emitted from the third LD 31C, is converged by a third collimator lens 53 and radiated toward a third irradiation range (see a sign 53A indicated by a broken line in Fig. 2 and a solid line in Fig. 3), and the green fluorescence is generated from the third irradiation range 53A.

The fourth LD 31D which is a fourth irradiation portion is provided facing the fourth wavelength conversion portion 38 positioned inside a first quadrant. The excitation light indicated by a solid line, which is emitted from the fourth LD 31D, is converged by a fourth collimator lens 54 and radiated toward a fourth irradiation range (see a sign 54A indicated by a broken line in Fig. 2 and a solid line in Fig. 3), and the umber fluorescence is generated from the third irradiation range 54A.

Note that the irradiation ranges 51A, 52A, 53A and 54A are circles of a same diameter dimension, and set larger than the width dimension w beforehand.

In the present embodiment, the first irradiation range 51A and the second irradiation range 52A, the second irradiation range 52A and the third irradiation range 53A, the third irradiation range 53A and the fourth irradiation range 54A, and the fourth irradiation range 54A and the first irradiation range 51A are position-shifted by about 90 degrees with the rotating shaft 32c as a center respectively.

Then, of the four irradiation ranges 51A, 52A, 53A and 54A, the first irradiation range 51A and the second irradiation range 52A, and the third irradiation range 53A and the fourth irradiation range 54A are provided in twos across the X axis 32X.

According to the configuration, since the irradiation ranges 51A, 52A, 53A and 54A are separated, when the excitation light is radiated from the LDs 31A, 31B, 31C and 31D toward the respective wavelength conversion portions 35, 36, 37 and 38 provided in the rotating body 32, the excitation light is prevented from being simultaneously radiated toward an almost same point of the rotating body 32.

Therefore, in the case that the excitation light is simultaneously radiated from the four LDs 31A, 31B, 31C and 31D, occurrence of a defect can be surely prevented, the defect being that a plurality of beams of the excitation light are simultaneously radiated to one of the phosphors causing sudden rise of a temperature and remarkable decline of conversion efficiency.

By radiation of violet or ultraviolet excitation light by the individual LDs 31A, 31B, 31C and 31D, the fluorescence generated from the phosphors of the individual wavelength conversion portions 35, 36, 37 and 38 is emitted toward a direction in which the excitation light is radiated.

Then, in emission directions of the wavelength conversion portions 35, 36, 37 and 38, the fluorescence pickup lenses 61, 62, 63 and 64 that function as converging lenses are provided respectively.

Specifically, optical axes of the individual fluorescence pickup lenses 61, 62, 63 and 64 and optical axes of the individual collimator lenses 51, 52, 53 and 54 are coaxially arranged.

Therefore, a first fluorescence pickup lens 55 is provided facing the first irradiation range 51A of the first wavelength conversion portion 35, a second fluorescence pickup lens 56 is provided facing the second irradiation range 52A of the second wavelength conversion portion 36, a third fluorescence pickup lens 57 is provided facing the third irradiation range 53A of the third wavelength conversion portion 37, and a fourth fluorescence pickup lens 58 is provided facing the fourth irradiation range 54A of the fourth wavelength conversion portion 38.

Then, between the individual fluorescence pickup lenses 61, 62, 63 and 64 and the individual collimator lenses 51, 52, 53 and 54, the dichroic filters 41, 42, 43 and 44 are arranged.

A first dichroic filter 41 arranged between the first collimator lens 51 and the first pickup lens 61 is an optical member having a characteristic of reflecting blue light which is the light of a specific wavelength and transmitting the light of the other wavelengths.

The first dichroic filter 41 is a multiplexing portion, and is inclined by a predetermined angle and arranged so as to reflect the blue fluorescence emitted from the first wavelength conversion portion 35 and converged by the first fluorescence pickup lens 61 toward a reflection mirror 70.

A second dichroic filter 42 arranged between the second collimator lens 52 and the second pickup lens 62 is an optical member having a characteristic of reflecting red light which is the light of a specific wavelength and transmitting the light of the other wavelengths.

The second dichroic filter 42 is a multiplexing portion, and is inclined by a predetermined angle and arranged at a predetermined position so as to reflect the red fluorescence emitted from the second wavelength conversion portion 36 and converged by the second fluorescence pickup lens 62 toward the reflection mirror 70.

Note that the reflection mirror 70 is also a multiplexing portion, and is inclined by a predetermined angle and arranged at a predetermined position so as to reflect the blue light and the red light made incident on the reflection mirror 70 toward a fifth dichroic filter 45 to be described later, which crosses the optical axis from a third dichroic filter 43 to an emission lens 81.

The third dichroic filter 43 arranged between the third collimator lens 53 and the third pickup lens 63 is an optical member having a characteristic of reflecting green light which is the light of a specific wavelength and transmitting the light of the other wavelengths.

The third dichroic filter 43 is a multiplexing portion, and is inclined by a predetermined angle and arranged at a predetermined position so as to reflect the green fluorescence emitted from the third wavelength conversion portion 37 and converged by the third fluorescence pickup lens 63 toward the emission lens 81.

A fourth dichroic filter 44 arranged between the fourth collimator lens 54 and the fourth pickup lens 64 is an optical member having a characteristic of reflecting umber light which is the light of a specific wavelength and transmitting the light of the other wavelengths.

The fourth dichroic filter 44 is a multiplexing portion, and is inclined by a predetermined angle and arranged at a predetermined position so as to reflect the umber fluorescence emitted from the fourth wavelength conversion portion 38 and converged by the fourth fluorescence pickup lens 64 toward the emission lens 81.

The fifth dichroic filter 45 is an optical member having a characteristic of reflecting the blue light and the red light which are the light of the specific wavelengths and transmitting the light of the other wavelengths, and is a multiplexing portion.

The fifth dichroic filter 45 is arranged between the fourth dichroic filter 44 and the emission lens 81, and is inclined by a predetermined angle and arranged at a predetermined position.

Note that the emission lens 81 is also one of the converging lenses, and radiates the light which passes through the lens 81 toward a proximal end face of the light guide fiber 10 disposed inside the light guide pipe sleeve 7a.

The blue fluorescence reflected at the first dichroic filter 41 and the red fluorescence reflected at the second dichroic filter 42 are reflected at the reflection mirror 70 and the fifth dichroic filter 45 and turn to the emission lens 81. On the other hand, the green fluorescence reflected at the third dichroic filter 43 and the umber fluorescence reflected at the fourth dichroic filter 44 are transmitted through the fifth dichroic filter 45 and turn to the emission lens 81.

In the present embodiment, the fourth irradiation range 54A is provided in the first quadrant for which the front surface 32f of the rotating body 32 is quadrisected by the X axis 32X and the Y axis 32Y, the third irradiation range 53A is provided in the second quadrant, the second irradiation range 52A is provided in the third quadrant, and the first irradiation range 51A is provided in the fourth quadrant. Then, the first fluorescence pickup lens 41 facing the first irradiation range 51A and the second fluorescence pickup lens 42 facing the second irradiation range 52A, and the third fluorescence pickup lens 43 facing the third irradiation range 53A and the fourth fluorescence pickup lens 44 facing the fourth irradiation range 54A are disposed across the X axis 32X.

Then, the first fluorescence pickup lens 41 and the second fluorescence pickup lens 42 are arranged at opposite positions across the Y axis 32Y. Therefore, mutual interference of the first fluorescence pickup lens 41 and the second fluorescence pickup lens 42 can be surely prevented.

Similarly, the third fluorescence pickup lens 43 and the fourth fluorescence pickup lens 44 are arranged at opposite positions across the Y axis 32Y. Therefore, mutual interference of the third fluorescence pickup lens 43 and the fourth fluorescence pickup lens 44 can be surely prevented.

Actions of the light source device 3 configured as described above will be described.

When performing the endoscope observation, a medical staff member operates an operation panel 85 and turns the light source device 3 to an ON state. Then, the motor drive signals are supplied from the control portion 34 to the motor 33, the rotating shaft 32c is rotated around the shaft at a predetermined rotation speed, and the rotating body 32 integrated with the rotating shaft 32c is rotated in a direction of an arrow Y in Fig. 1.

In addition, the driving current is supplied from the control portion 34 to the LDs 31 A, 31B, 31C and 31D, and the excitation light is emitted from the individual LDs 31A, 31B, 31C and 31D to the corresponding collimator lenses 51, 52, 53 and 54.

Then, the excitation light converged at the first collimator lens 51 is radiated toward the first irradiation range 51A of the front surface 32f, the excitation light converged at the second collimator lens 52 is radiated toward the second irradiation range 52A, the excitation light converged at the third collimator lens 53 is radiated toward the third irradiation range 53A, and the excitation light converged at the fourth collimator lens 54 is radiated toward the fourth irradiation range 54A.

Then, the blue fluorescence is emitted from the first irradiation range 51A of the first wavelength conversion portion 35 irradiated with the excitation light, the red fluorescence is emitted from the second irradiation range 52A of the second wavelength conversion portion 36 irradiated with the excitation light, the green fluorescence is emitted from the third irradiation range 53A of the third wavelength conversion portion 37 irradiated with the excitation light, and the umber fluorescence is emitted from the fourth irradiation range 54A of the fourth wavelength conversion portion 38 irradiated with the excitation light.

At the time, since the rotating body 32 is rotated by the motor 33, the excitation light is not continuously radiated to a part of the phosphors of the wavelength conversion portions 35, 36, 37 and 38 formed in an annular shape, but is cyclically radiated to the phosphors provided in the annular areas CA1, CA2, CA3 and CA4 of the width w of the wavelength conversion portions 35, 36, 37 and 38 that are rotationally moved.

As a result, the rotationally moved annular phosphors receive the excitation light and generate the fluorescence only when passing through the irradiation range. Then, the annular phosphors do not receive the excitation light while being rotationally moved outside the irradiation range. Therefore, temperature dissipation due to rise of a temperature of the phosphors is avoided, and a defect that a light quantity emitted from the phosphors declines can be prevented.

In addition, between the phosphor in the annular area CA4 provided on an outer peripheral side of the rotating body 32 and the phosphor in the annular area CA1 provided on a center side, an irradiation area (irradiation moving area) per unit time period is larger for the phosphor provided on the outer peripheral side for the greater radius, and generated heat is dispersed in a wide range.

Thus, by providing the fourth phosphor and the third phosphor having a characteristic that conversion efficiency of the wavelength easily declines due to the rise of the temperature among the four phosphors in the fourth wavelength conversion portion 38 and the third wavelength conversion portion 37, the decline of the conversion efficiency due to the rise of the temperature of the phosphors can be effectively prevented.

The fluorescence emitted from the individual wavelength conversion portions 35, 36, 37 and 38 is converged at the individual fluorescence pickup lenses 61, 62, 63 and 64 as indicated by two-dot chain lines, and reflected at the individual dichroic filters 41, 42, 43 and 44 thereafter.

Then, as described above, the blue fluorescence reflected at the first dichroic filter 41 and the red fluorescence reflected at the second dichroic filter 42 are reflected at the reflection mirror 70 and the fifth dichroic filter 45, then converged at the emission lens 81, and radiated to the proximal end face of the light guide fiber 10.

On the other hand, the green fluorescence reflected at the third dichroic filter 43 and the umber fluorescence reflected at the fourth dichroic filter 44 are transmitted through the fifth dichroic filter 45, then converged at the emission lens 81, and radiated to the proximal end face of the light guide fiber 10.

Each fluorescence made incident from the proximal end face of the light guide fiber 10 is transmitted inside the light guide fiber 10, passes through the illumination lens 9a, and is emitted toward a target part. As a result, the target part is illuminated by illumination light suitable for the endoscope observation.

In this way, in the four annular areas CA1, CA2, CA3 and CA4 provided on the front surface 32f of one rotating body 32, the first phosphor, the second phosphor, the third phosphor and the fourth phosphor are provided, and the first wavelength conversion portion 35, the second wavelength conversion portion 36, the third wavelength conversion portion 37 and the fourth wavelength conversion portion 38 are provided. As a result, by rotating one rotating body 32 by one motor 33, the number of components is reduced, and miniaturization of the device can be realized.

In addition, the irradiation ranges of the excitation light are set so as not to overlap inside the rotating body 32. As a result, simultaneous radiation of the plurality of beams of the excitation light to a predetermined irradiation range of one phosphor causing the sudden rise of the temperature of the phosphor can be surely prevented.

In addition, the front surface 32f of the rotating body 32 is quadrisected as described above, and one of the irradiation ranges 54A, 53A, 52A and 51A is provided in each quadrant. In addition, the first fluorescence pickup lens 41 and the second fluorescence pickup lens 42, and the third fluorescence pickup lens 43 and the fourth fluorescence pickup lens 44 are disposed across the X axis 32X, the first fluorescence pickup lens 41 and the second fluorescence pickup lens 42 are arranged at the opposite positions across the Y axis 32Y, and the third fluorescence pickup lens 43 and the fourth fluorescence pickup lens 44 are arranged at the opposite positions across the Y axis 32Y.

As a result, while preventing the mutual interference of the fluorescence pickup lenses 41, 42, 43 and 44 with each other, the fluorescence emitted from the individual phosphors can be efficiently converged.

Note that, in the light source device 3 described above, the four wavelength conversion portions are provided on the front surface 32f of the rotating body 32, the front surface 32f of the rotating body 32 is quadrisected, and one irradiation range and one pickup lens are provided inside each divided range.

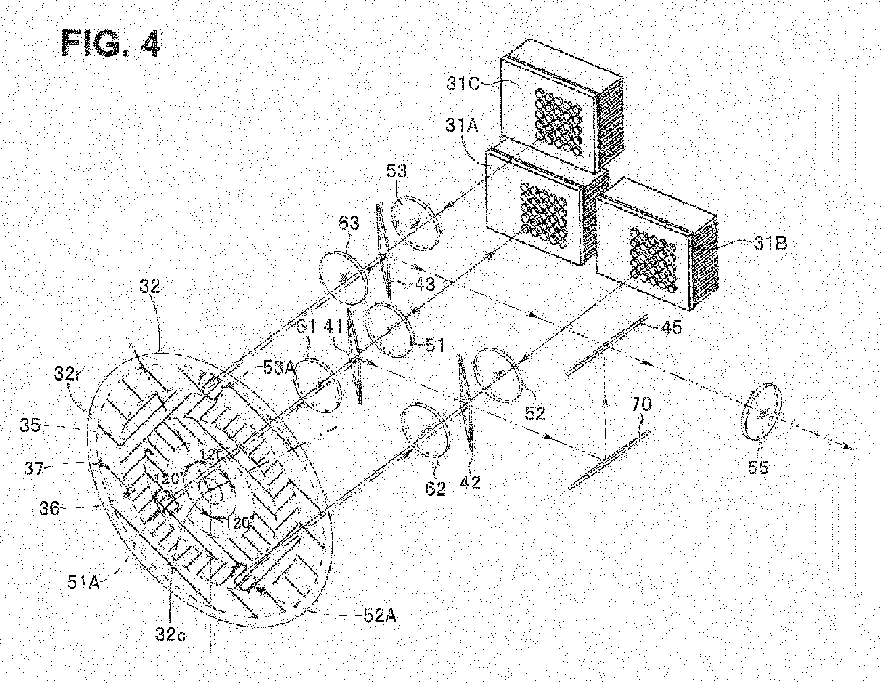

However, two or three, as illustrated in Fig. 4, wavelength conversion portions may be provided on the front surface 32f of the rotating body 32, or five or more wavelength conversion portions may be provided on the front surface 32f of the rotating body 32.

Then, The front surface 32f is bisected in the case of providing two wavelength conversion portions on the front surface 32f of the rotating body 32, the front surface 32f is trisected in the case of providing three wavelength conversion portions on the front surface 32f of the rotating body 32 as illustrated in Fig. 4, the front surface 32f is divided according to the number of the wavelength conversion portions in the case of providing five or more wavelength conversion portions on the front surface 32f of the rotating body 32, and one irradiation range and one pickup lens are provided inside each divided range.

In addition, the width of the annular areas CA1, CA2, CA3 and CA4 is turned to w. However, the width of the annular areas CA1, CA2, CA3 and CA4 may be adjusted in consideration of light emitting efficiency.

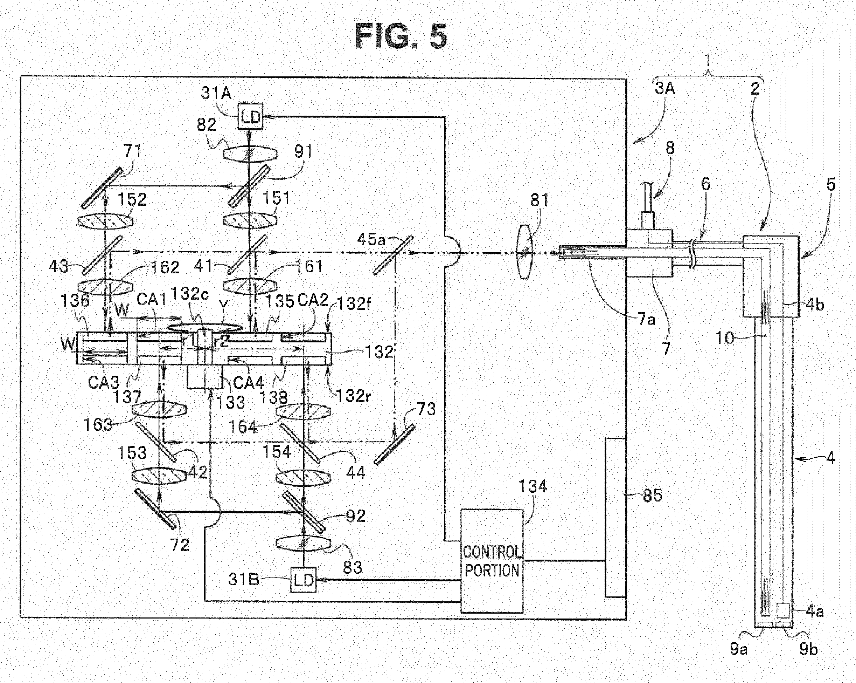

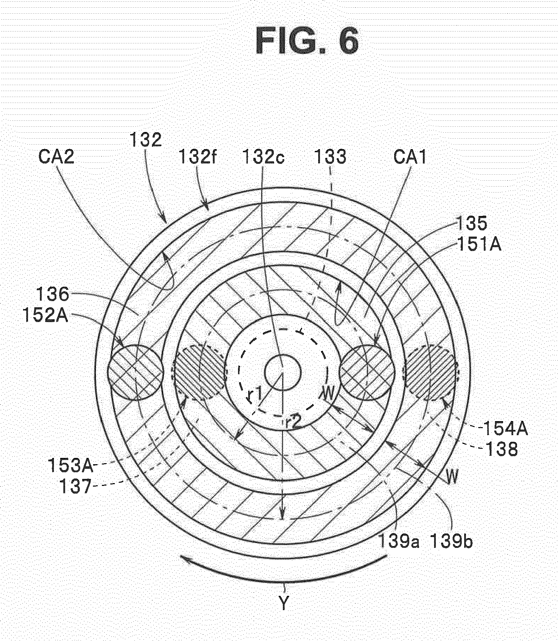

A second embodiment of the light source device 3 will be described with reference to Fig. 5 and Fig. 6.

Note that the same signs are attached to same members as the embodiments described above, and the descriptions are omitted.

As illustrated in Fig. 5, a light source device 3A is configured mainly including the LDs 31A and 31B, a rotating body 132, the motor 33, the control portion 34, a plurality of half mirrors 91 and 92, a plurality of converging lenses 151-154, a plurality of reflection mirrors 71-73, a plurality of fluorescence pickup lenses 161-164, and a plurality of dichroic filters 41-45a.

Note that, in the present embodiment, the first phosphor receives the excitation light and generates the blue fluorescence, the second phosphor receives the excitation light and generates the red fluorescence, the third phosphor receives the excitation light and generates the green fluorescence, and the fourth phosphor receives the excitation light and generates the umber fluorescence.

In the present embodiment, the two LDs 31A and 31B are prepared as light sources. Then, the rotating body 132 is, in the present embodiment, provided with a front side LD 31A on a front surface 32f side of the rotating body 132, and provided with a rear side LD 31 B on a rear surface 32r side.

As illustrated in Fig. 5 and Fig. 6, two kinds of wavelength conversion portions 135 and 136 are provided on a front surface 132f of the rotating body 132, and two kinds of wavelength conversion portions 137 and 138 are also provided on a rear surface 132r. The first wavelength conversion portion 135, the second wavelength conversion portion 136, the third wavelength conversion portion 137 and the fourth wavelength conversion portion 138 are configured including the phosphors. The first wavelength conversion portion 135, the second wavelength conversion portion 136, the third wavelength conversion portion 137, and the fourth wavelength conversion portion 138 receive the light of the LDs 31A and 31B, and emit the fluorescence of the respectively different wavelengths that realize the color reproducibility suitable for the endoscope observation.

As illustrated in Fig. 6, the first wavelength conversion portion 135 is a first front surface side wavelength conversion portion, and is configured including the first phosphor in the first annular area CA1 formed along a first circle 139a formed on the center side of the front surface 132f with the radius r1 from the center of a rotating shaft 132c. The width of the first annular area CA1 is w, and the first wavelength conversion portion 135 is an annular portion provided on the front surface 132f having the width w with a circumference of the first circle 139a as the center line.

In contrast, the second wavelength conversion portion 136 is a second front surface side wavelength conversion portion, and is configured including the third phosphor in the second annular area CA2 of the width w formed along a second circle 139b formed on the outer peripheral side of the front surface 132f with the radius r2 from the center of the rotating shaft 132c. That is, the second wavelength conversion portion 136 is an annular portion provided on the front surface 132f having the width w with a circumference of the second circle 139b as the center line.

The first wavelength conversion portion 135 and the second wavelength conversion portion 136 are provided separately so as not to be arranged overlapping with each other on the front surface 132f.

On the other hand, the third wavelength conversion portion 137 is a first rear surface side wavelength conversion portion, and is configured including the second phosphor in the third annular area CA3 of the width w formed along the first circle 139a formed with the radius r1 on the rear surface 132r. That is, the third wavelength conversion portion 137 is an annular portion provided on the rear surface 132r having the width w with the circumference of the first circle 139a as the center line.

The fourth wavelength conversion portion 138 is a second rear surface side wavelength conversion portion, and is configured including the fourth phosphor in the fourth annular area CA4 of the width w formed along the second circle 139b formed with the radius r2 on the rear surface 132r. That is, the fourth wavelength conversion portion 138 is an annular portion provided on the rear surface 132r having the width w with the circumference of the second circle 139b as the center line.

The third wavelength conversion portion 137 and the fourth wavelength conversion portion 138 are provided separately as described above on the rear surface 132r.

That is, the third wavelength conversion portion 137 is provided on the opposite surface of the first wavelength conversion portion 135, and the fourth wavelength conversion portion 138 is provided on the opposite surface of the second wavelength conversion portion 136. In other words, on the rotating body 132, the first wavelength conversion portion 135 and the third wavelength conversion portion 137 are arranged overlapping with each other, and the second wavelength conversion portion 136 and the fourth wavelength conversion portion 138 are arranged overlapping with each other.

The control portion 134 supplies the motor drive signals to the motor 133 to control the rotation speed of the motor 133, and supplies the driving current to the LDs 31A and 31B to adjust the emission light quantities from the LDs 31A and 31B.

For the excitation light indicated by a solid line, which is emitted from the front side LD 31A, a luminous flux is divided into two by the front side half mirror 91 as illustrated in Fig. 1.

The light transmitted through the front side half mirror 91 is converged by a first converging lens 151 and radiated toward the first irradiation range (see a sign 51A indicated by a solid line in Fig. 6) of the front surface 132f. The first converging lens 151 is a first front surface side irradiation portion, and is provided facing the first wavelength conversion portion 135. Therefore, the blue fluorescence is generated from the first irradiation range 151A irradiated with the excitation light of the first wavelength conversion portion 135 illustrated in Fig. 6.

In contrast, the light reflected at the front side half mirror 91 illustrated in Fig. 1 is further reflected at a first reflection mirror 71, converged at a second converging lens 152 thereafter, and radiated toward the second irradiation range (see a sign 152A indicated by a solid line in Fig. 2) of the front surface 132f. The second converging lens 152 is a second front surface side irradiation portion, and is provided facing the second wavelength conversion portion 136. Therefore, the green fluorescence is generated from the second irradiation range 152A irradiated with the excitation light of the second wavelength conversion portion 136 illustrated in Fig. 2.

On the other hand, for the excitation light emitted from the rear side LD 31 B, the luminous flux is divided into two by the rear side half mirror 92.

The light transmitted through the rear side half mirror 92 turns to a fourth converging lens 154, is converged at the lens 154, and is emitted toward the fourth irradiation range (see 54A indicated by a broken line in Fig. 6) of the rear surface 132r. The fourth converging lens 154 is a second rear surface side irradiation portion, and is provided facing the fourth wavelength conversion portion 138. Therefore, the umber fluorescence is generated from the fourth irradiation range 154A irradiated with the excitation light of the fourth wavelength conversion portion 138 illustrated in Fig. 6.

In contrast, the light reflected at the rear side half mirror 92 illustrated in Fig. 5 is further reflected at a second reflection mirror 72, converged at a third converging lens 153 thereafter, and radiated toward the third irradiation range (see 153A indicated by a broken line in Fig. 6) of the rear surface 132r. The third converging lens 153 is a first rear surface side irradiation portion, and is provided facing the third wavelength conversion portion 137. Therefore, the red fluorescence is generated from the third irradiation range 153A irradiated with the excitation light of the third wavelength conversion portion 137 illustrated in Fig. 6.

Note that the irradiation ranges 151A, 152A, 153A and 154A are circles of a same diameter dimension, and are set larger than the width dimension w beforehand.

In the present embodiment, the first irradiation range 151A of the first converging lens 151 and the second irradiation range 152A of the second converging lens 152 are set in different areas across a line segment passing through the rotating shaft 132c as illustrated in Fig. 2.

In addition, the third irradiation range 153A of the third converging lens 153 and the fourth irradiation range 154A of the fourth converging lens 154 are set in the different areas across the line segment passing through the rotating shaft 132c.

Then, the third irradiation range 153A of the third converging lens 153 is set at a position different from the opposite surface side of the first irradiation range 151A of the first converging lens 151, and the fourth irradiation range 154A of the fourth converging lens 154 is set at a position different from the opposite surface side of the third irradiation range 153A of the third converging lens 153.

Specifically, in the present embodiment, as illustrated in Fig. 6, the first irradiation range 151A positioned on the center side of the front surface 132f and the second irradiation range 152A positioned on the outer peripheral side of the front surface 132f are position-shifted by 180 degrees across the rotating shaft 132c. In addition, the third irradiation range 153A positioned on the center side of the rear surface 132r and the fourth irradiation range 154A positioned on the outer peripheral side of the rear surface 132r are position-shifted by 180 degrees across the rotating shaft 132c.

Therefore, the two converging lens irradiation ranges 151A and 152A provided on the front surface 132f of the rotating body 132 and the two converging lens irradiation ranges 153A and 154A provided on the rear surface 132r are separated so as not to overlap in a view from one surface side as illustrated in Fig. 2.

According to the configuration, since the irradiation ranges 151A, 152A, 153A and 154A are separated, when the excitation light is radiated toward the respective wavelength conversion portions 135, 136, 137 and 138 provided in the rotating body 132, the excitation light is prevented from being simultaneously radiated toward the almost same point on the front surface 132f and the rear surface 132r of the rotating body 132. As a result, the occurrence of a defect can be surely prevented, the defect being that one part is irradiated with the excitation light simultaneously from two directions and the temperature of the phosphor suddenly rises, thereby causing the remarkable decline of the conversion efficiency.

Note that the first reflection mirror 71 is inclined by a predetermined angle and arranged at a predetermined position such that the light made incident on the reflection mirror 71 is emitted toward the second converging lens 152. In addition, the second reflection mirror 72 is inclined by a predetermined angle and arranged at a predetermined position such that the light made incident on the reflection mirror 72 is emitted toward the third converging lens 153.

The fluorescence generated from the phosphors of the individual wavelength conversion portions 135, 136, 137 and 138 by the radiation of the violet or ultraviolet excitation light is emitted toward the direction in which the excitation light is radiated.

Then, in emission directions of the individual wavelength conversion portions 135, 136, 137 and 138, the fluorescence pickup lenses 161, 162, 163 and 164 that function as the converging lenses are provided respectively.

Specifically, the optical axes of the individual fluorescence pickup lenses 161, 162, 163 and 164 and the optical axes of the individual converging lenses 151, 152, 153 and 154 are coaxially arranged.

Therefore, a first fluorescence pickup lens 161 is provided facing the first irradiation range 151A of the first wavelength conversion portion 135, a second fluorescence pickup lens 162 is provided facing the second irradiation range 152A of the second wavelength conversion portion 136, a third fluorescence pickup lens 163 is provided facing the third irradiation range 153A of the third wavelength conversion portion 137, and a fourth fluorescence pickup lens 164 is provided facing the fourth irradiation range 154A of the fourth wavelength conversion portion 138.

The first dichroic filter 41 is arranged between the first converging lens 151 and the first wavelength conversion portion 135 provided on the front surface 132f side of the rotating body 132. The first dichroic filter 41 is a multiplexing portion, and is inclined by a predetermined angle and arranged so as to reflect the blue fluorescence emitted from the first wavelength conversion portion 135 and converged by the first fluorescence pickup lens 61 toward the emission lens 81.

The second dichroic filter 42 is arranged between the third converging lens 153 and the third wavelength conversion portion 137 provided on the rear surface 132r side of the rotating body 132. The second dichroic filter 42 is a multiplexing portion, and is inclined by a predetermined angle and arranged at a predetermined position so as to reflect the red fluorescence emitted from the third wavelength conversion portion 136 and converged by the third fluorescence pickup lens 163 toward the reflection mirror 73.

The third dichroic filter 43 is arranged between the second converging lens 152 and the second wavelength conversion portion 136 provided on the front surface 132f side of the rotating body 132. The third dichroic filter 43 is a multiplexing portion, and is inclined by a predetermined angle and arranged at a predetermined position so as to reflect the green fluorescence emitted from the second wavelength conversion portion 137 and converged by the second fluorescence pickup lens 162 toward the emission lens 81.

Note that a third reflection mirror 73 is also a multiplexing portion, and is inclined by a predetermined angle and arranged at a predetermined position such that the light made incident on the reflection mirror 73 crosses the optical axis from the first dichroic filter 41 to the emission lens 81.

The fourth dichroic filter 44 is arranged between the fourth converging lens 154 and the fourth wavelength conversion portion 138 provided on the rear surface 132r side of the rotating body 132. The fourth dichroic filter 44 is a multiplexing portion, and is inclined by a predetermined angle and arranged at a predetermined position so as to reflect the umber fluorescence emitted from the fourth wavelength conversion portion 138 and converged by the fourth fluorescence pickup lens 164 toward the third reflection mirror 73.

A fifth dichroic filter 45a is an optical member having a characteristic of reflecting the red light and the umber light which are the light of the specific wavelengths and transmitting the light of the other wavelengths, and is a multiplexing portion.

The fifth dichroic filter 45a is arranged between the first dichroic filter 41 and the emission lens 81, and is inclined by a predetermined angle and arranged at a predetermined position.

The blue fluorescence reflected at the first dichroic filter 41 and the green fluorescence reflected at the third dichroic filter 43 are transmitted through the fifth dichroic filter 45a and turn to the emission lens 81. On the other hand, the red fluorescence and the umber fluorescence reflected at the reflection mirror 73 are reflected at the fifth dichroic filter 45 a and turn to the emission lens 81.

In the present embodiment, the first irradiation range 151A and the second irradiation range 152A positioned on the front surface 132f side are position-shifted by 180 degrees across the rotating shaft 132c. Then, the first fluorescence pickup lens 161 is made to face the first irradiation range 151A in the first wavelength conversion portion 135, and the second fluorescence pickup lens 162 is made to face the second irradiation range 152A in the second wavelength conversion portion 135.

As a result, the first fluorescence pickup lens 161 and the second fluorescence pickup lens 162 are arranged at opposite positions across the rotating shaft 132c. Thus, the mutual interference of the first fluorescence pickup lens 161 and the second fluorescence pickup lens 162 can be surely prevented.

Similarly, the third fluorescence pickup lens 1637 and the fourth fluorescence pickup lens 164 arranged on the rear surface 132r side are arranged at opposite positions across the rotating shaft 132c. Thus, the mutual interference of the third fluorescence pickup lens 163 and the fourth fluorescence pickup lens 164 can be surely prevented.

Note that a sign 82 denotes a front side converging lens, and converges the light emitted from the front side LD 31A to the front side half mirror 91. A sign 83 denotes a rear side converging lens, and converges the light emitted from the rear side LD 31B to the rear side half mirror 92. The sign 85 is the operation panel.

Actions of the light source device 3A configured as described above will be described.

When performing the endoscope observation, a medical staff member operates the operation panel 85 and turns the light source device 3A to an ON state. Then, the motor drive signals are supplied from the control portion 134 to the motor 133, the rotating shaft 132c is rotated around the shaft at a predetermined rotation speed, and the rotating body 32 integrated with the rotating shaft 132c is rotated in a direction of an arrow Y in Fig. 5 and Fig. 6.

In addition, the driving current is supplied from the control portion 134 to the LDs 31A and 31B, the excitation light is emitted from the front side LD 31A to the front side half mirror 91, and the excitation light is emitted from the rear side LD 31B to the rear side half mirror 92.

The excitation light respectively emitted from the LDs 31A and 31B is bisected at the half mirrors 91 and 92 as described above.

One of the excitation light divided at the front side half mirror 91 is converged by the first converging lens 151 and radiated toward the first irradiation range 151A of the front surface 132f. The other excitation light is converged by the second converging lens 152 and radiated toward the second irradiation range 152A of the front surface 132f.

On the other hand, one of the excitation light divided at the rear side half mirror 92 is converged by the third converging lens 153 and radiated toward the third irradiation range 153A of the rear surface 132r. The other excitation light is converged by the fourth converging lens 154 and radiated toward the fourth irradiation range 154A of the rear surface 132r.

Then, the blue fluorescence is emitted from the first wavelength conversion portion 135 irradiated with the excitation light, the green fluorescence is emitted from the second wavelength conversion portion 136 irradiated with the excitation light, the red fluorescence is emitted from the third wavelength conversion portion 137 irradiated with the excitation light, and the umber fluorescence is emitted from the fourth wavelength conversion portion 138 irradiated with the excitation light.

At the time, since the rotating body 132 is rotated by the motor 133, the excitation light is not continuously radiated to a part of the phosphors provided in the wavelength conversion portions 135, 136, 137 and 138 formed in the annular shape, but is radiated to the entire periphery of the phosphors of the wavelength conversion portions 135, 136, 137 and 138 that are rotationally moved.

As a result, the rotationally moved annular phosphors receive the excitation light and generate the fluorescence only when passing through the irradiation range, and do not receive the excitation light while being rotationally moved outside the irradiation range. Therefore, the temperature dissipation due to the rise of the temperature of the phosphors is avoided, and the defect that the light quantity emitted from the phosphors declines can be prevented.

In addition, between the annular phosphor provided on the outer peripheral side and the annular phosphor provided on the center side, the irradiation area (irradiation moving area) per unit time period is larger for the phosphor provided on the outer peripheral side for the greater radius, and the generated heat is dispersed in a wide range.

Thus, among the four phosphors, by providing the third phosphor having a characteristic that the conversion efficiency of the wavelength easily declines due to the rise of the temperature in the second wavelength conversion portion 136 of the front surface outer peripheral side and providing the fourth phosphor in the fourth wavelength conversion portion 138 of the rear surface outer peripheral side, the decline of the conversion efficiency due to the rise of the temperature of the phosphors can be prevented.

The fluorescence emitted from the individual wavelength conversion portions 135, 136, 137 and 138 is converged at the individual fluorescence pickup lenses 161, 162, 163 and 164 as indicated by two-dot chain lines, and reflected at the individual dichroic filters 41, 42, 43 and 44 thereafter.

Then, as described above, the blue fluorescence reflected at the first dichroic filter 41 and the green fluorescence reflected at the second dichroic filter 43 are transmitted through the fifth dichroic filter 45 a, then converged at the emission lens 81, and radiated to the proximal end face of the light guide fiber 10. The red fluorescence reflected at the third dichroic filter 42 and the umber fluorescence reflected at the fourth dichroic filter 44 are reflected at the third reflection mirror 73, reflected at the fifth dichroic filter 45a thereafter, then converged at the emission lens 81, and radiated to the proximal end face of the light guide fiber 10.

Each fluorescence made incident from the proximal end face of the light guide fiber 10 is transmitted inside the light guide fiber 10, passes through the illumination lens 9a, and is emitted toward the target part. As a result, the target part is illuminated by the illumination light suitable for the endoscope observation.

In this way, while the first wavelength conversion portion 135 including the first phosphor and the second wavelength conversion portion 136 including the third phosphor are provided on the front surface 132f of one rotating body 132, the third wavelength conversion portion 137 including the second phosphor and the fourth wavelength conversion portion 138 including the fourth phosphor are provided on the rear surface 132r.

As a result, by rotating one rotating body 132 by one motor 133 similarly to the description above, the number of components is reduced, and miniaturization of the device can be realized. In addition, for an area of the rotating body 132 provided with two each of the four wavelength conversion portions on both surfaces, the diameter becomes smaller compared to the rotating body 32 provided with the four wavelength conversion portions on one surface, and the miniaturization of the light source device 3A can be realized.

In addition, the irradiation ranges of the excitation light are set so as not to overlap inside the rotating body. As a result, the simultaneous radiation of the plurality of beams of the excitation light to a predetermined irradiation range of one phosphor causing the sudden rise of the temperature of the phosphor can be surely prevented.

In addition, by setting arrangement positions of the fluorescence pickup lenses provided facing the two wavelength conversion portions on the front surface side and the rear surface side based on the irradiation ranges set in the different areas across the line segment passing through the rotating shaft 132c, while preventing the interference of the fluorescence pickup lenses with each other, the fluorescence emitted from the individual phosphors can be efficiently converged.

Note that, in the light source device 3A described above, the front side LD 31A and the rear side LD 31B are provided. However, the configuration may be such that only one LD 31 is provided, and the excitation light emitted from the LD 31 may be divided into four and supplied to the individual converging lenses 151-154.

In addition, in the above-described embodiment, the two irradiation ranges 151A and 52A on the front surface 132f and the two irradiation ranges 153A and 54A on the rear surface 132r are position-shifted by 180 degrees across the rotating shaft 132c respectively. However, an angle of position shift is not limited to 180 degrees as long as the interference of the two fluorescence pickup lenses 161 and 162 arranged facing the irradiation ranges 151A and 152A with each other and the interference of the two fluorescence pickup lenses 163 and 164 arranged facing the irradiation ranges 153A and 154A with each other can be prevented.

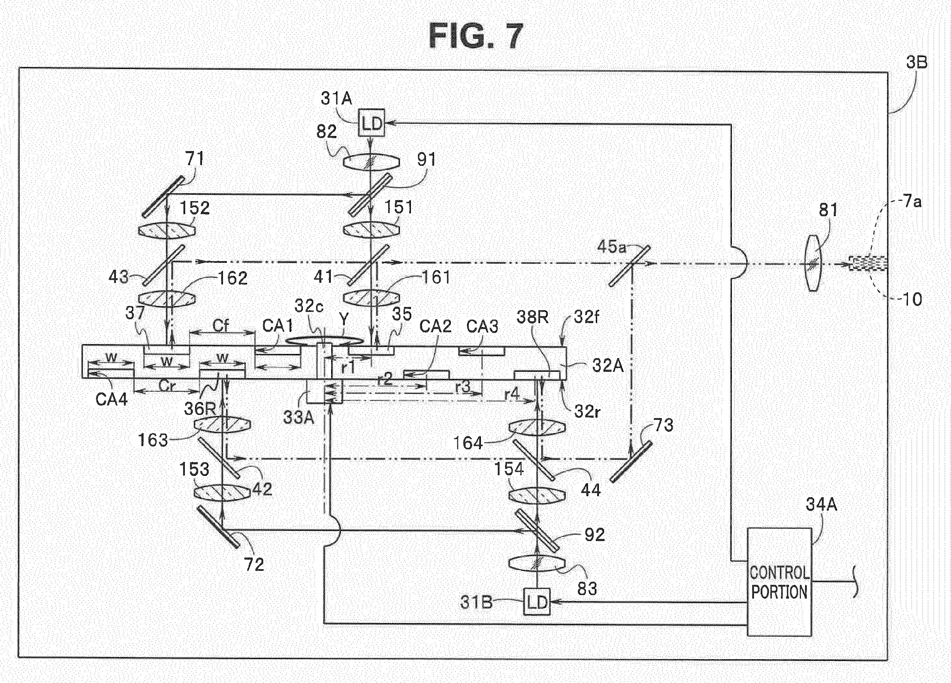

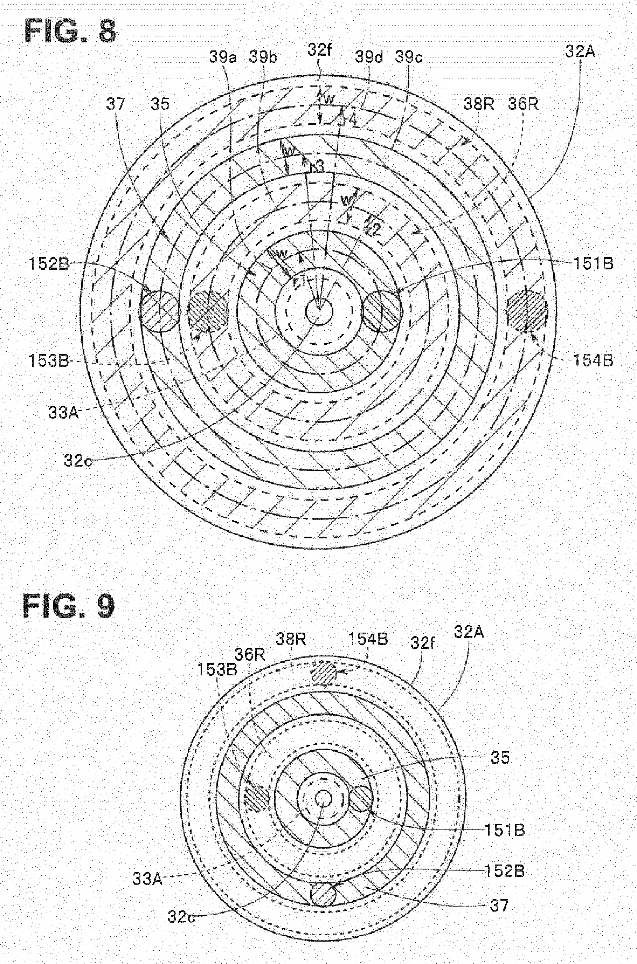

A modification of a light source device 3B will be described with reference to Fig. 7 and Fig. 8.

As illustrated in Fig. 7, the light source device 3B is configured mainly including the front side LD 31A and the rear side LD 31B, a rotating body 32A, a motor 33A, a control portion 34A, the plurality of half mirrors 91 and 92, the plurality of converging lenses 151-154, the plurality of reflection mirrors 71-73, the plurality of fluorescence pickup lenses 161-164, and the plurality of dichroic filters 41-45a.

In the present embodiment, the rotating body 32A is configured almost same as the rotating body 32 in the first embodiment, and the rotating body 132A is rotationally driven by the motor 33A. The other components of the light source device 3B are similar to those of the embodiments described above, the same signs are attached to same members, and the descriptions are omitted.

As illustrated in Fig. 7 and Fig. 8, two kinds of the wavelength conversion portions 35 and 37 are provided on the front surface 32f of the rotating body 32A in the present embodiment, and two kinds of wavelength conversion portions 36R and 38R are provided on the rear surface 32r. That is, the rotating body 32 and the rotating body 32A are different in a point that the second wavelength conversion portion 36R and the fourth wavelength conversion portion 38R are provided on the rear surface 32r.

Then, the first wavelength conversion portion 35, the second wavelength conversion portion 36R, the third wavelength conversion portion 37, and the fourth wavelength conversion portion 37R are configured including the phosphors similarly to the description above.

In the present embodiment, the second wavelength conversion portion 36R is configured including the second phosphor in the second annular area CA2 of the width w formed along the second circle 39b formed on the rear surface with the radius r2 from the center of the rotating shaft 32c.

The fourth wavelength conversion portion 38R is configured including the fourth phosphor in the fourth annular area CA4 of the width w formed along the fourth circle 39d formed on the outer peripheral side of the rear surface 32r with the radius r4 from the center of the rotating shaft 32c.

In the present embodiment, the second wavelength conversion portion 36R is provided on the opposite surface of a front side clearance Cf formed between the first wavelength conversion portion 35 and the third wavelength conversion portion 37. In addition, the third wavelength conversion portion 37 is provided on the opposite surface of a rear side clearance Cr formed between the second wavelength conversion portion 36R and the fourth wavelength conversion portion 38R.

Then, in the front view of the rotating body 32A from the front surface 32f side, the four of the first wavelength conversion portion 35 indicated by a solid line in Fig. 8, the second wavelength conversion portion 36R indicated by a broken line, the third wavelength conversion portion 37 indicated by a solid line, and the fourth wavelength conversion portion 38R indicated by a broken line are separated without overlapping, and concentrically arrayed with the rotating shaft 32c as the center.

Therefore, irradiation ranges 151B and 152B of the two converging lenses 151 and 152 provided on the front surface 32f side of the rotating body 32A and irradiation ranges 153B and 154B of the two converging lenses 153 and 154 provided on the rear surface 32r side are separated without overlapping in a view from one surface side as illustrated in Fig. 8.

According to the configuration, the irradiation ranges 151B, 152B, 153B and 154B are provided separately without overlapping in the view from one surface side. Thus, when the excitation light is radiated toward the respective wavelength conversion portions 35, 36R, 37 and 38R provided in the rotating body 32A, the defect that the excitation light is simultaneously radiated toward the almost same point on the front surface 32f and the rear surface 32r of the rotating body 32 can be dissolved.

In addition, for the greater radii of the third wavelength conversion portion 37 provided on the outer peripheral side of the front surface 32f and the fourth wavelength conversion portion 38A provided on the outer peripheral side of the rear surface 32r, the irradiation area (irradiation moving area) per unit time period is larger compared to the above-described embodiments so that the decline of the conversion efficiency due to the rise of the temperature of the phosphor can be more surely prevented. In other words, it becomes possible to provide the phosphor having the characteristic that conversion efficiency of the wavelength easily declines due to the rise of the temperature in the third wavelength conversion portion 37 and the fourth wavelength conversion portion 38R, and a selection range of the phosphor can be widened.

The other actions and effects are similar to those of the above-described embodiments.

Note that the irradiation ranges 151B, 152B, 153B and 154B are not limited to the positions illustrated in Fig. 8, and by position-shifting the first irradiation range 151B and the second irradiation range 152B indicated by solid lines by 90 degrees for example and position-shifting the third irradiation range 153B and the fourth irradiation range 154B indicated by broken lines by 90 degrees for example as illustrated in Fig. 9, the irradiation ranges 151B, 152B, 153B and 154B maybe provided separately without overlapping and without being adjacent in the view from one surface side.

As a result, a distance between the irradiation ranges 151B and 152B on the front surface 132f and the irradiation ranges 153B and 154B on the rear surface 132r is widened and the temperature rise of the phosphor can be more surely prevented.

Note that the angle of the position shift is not limited to 90 degrees, and may be equal to or larger than 90 degrees or smaller than 90 degrees as long as the interference of the pickup lenses with each other can be prevented. In addition, the interference of the pickup lenses with each other may be prevented by appropriately adjusting the position shift angle, the front side clearance Cf and the rear side clearance Cr.

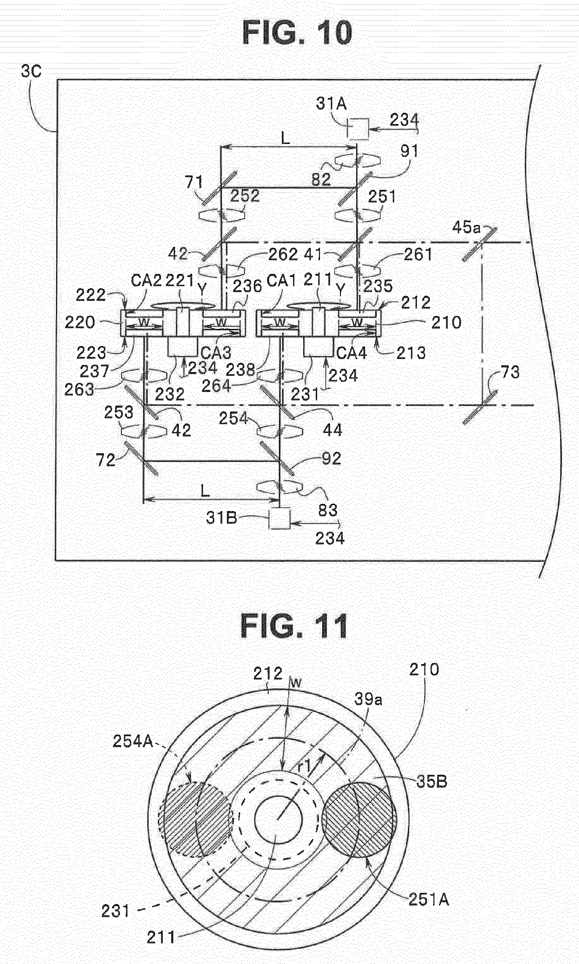

A different configuration example of the light source device will be described with reference to Fig. 10 to Fig. 13.

A light source device 3C illustrated in Fig. 10 is configured mainly including the front side LD 31A and the rear side LD 31B, two rotating bodies 210 and 220, motors 231 and 232, a control portion (not shown in the figure), the plurality of half mirrors 91 and 92, a plurality of converging lenses 251-254, the plurality of reflection mirrors 71-73, a plurality of fluorescence pickup lenses 261-264, and the plurality of dichroic filters 41-45a.

That is, in the present embodiment, the two rotating bodies 210 and 220 are provided, and a first motor 231 that rotationally drives a first rotating body 210 and a second motor 232 that rotationally drives a second rotating body 220 are provided. The other components are similar to those of the embodiments described above, the same signs are attached to the same members, and the descriptions are omitted.

The first rotating body 210 and the second rotating body 220 are in the almost similar configuration, and are planar disks. On the center positions of the respective rotating bodies 210 and 220, rotating shafts 211 and 221 are integrally provided. The respective rotating shafts 211 and 221 are provided with the motors 231 and 232 respectively.

A first wavelength conversion portion 235 is provided on a front surface 212 of the first rotating body 210 as illustrated in Fig. 10 and Fig. 11, and a second wavelength conversion portion 236 is provided on a front surface 212 of the second rotating body 220 as illustrated in Fig. 10. Then, a third wavelength conversion portion 237 is provided on a rear surface 213 of the second rotating body 220, and a fourth wavelength conversion portion 38B is provided on a rear surface 213r of the first rotating body 210.

The front side LD 31A is provided on the side of the front surfaces 212 and 222 of the rotating bodies 210 and 220, and the rear side LD 31B is provided on the side of the rear surfaces 213 and 223.

As illustrated in Fig. 11, the first wavelength conversion portion 235 is configured including the first phosphor in the first annular area CA1 formed along the first circle 39a formed on the front surface 212 of the first rotating body 210 with the radius r1 from the center of the rotating shaft 211. The width of the first annular area CA1 is w for example.

In contrast, the second wavelength conversion portion 236 is configured including the third phosphor in the second annular area CA2 of the width w formed along a first circle (not shown in the figure) formed on the front surface 222 of the second rotating body 220 with the radius r1 from the center of the rotating shaft 221.

In addition, the third wavelength conversion portion 237 is configured including the second phosphor in the third annular area CA3 of the width w formed along the first circle (not shown in the figure) formed on the rear surface 223 of the second rotating body 220 with the radius r1 from the center of the rotating shaft 221.

In addition, the fourth wavelength conversion portion 238 is configured including the fourth phosphor in the fourth annular area CA4 of the width w formed along the first circle 39a formed on the rear surface 212 of the first rotating body 210 with the radius r1 from the center of the rotating shaft 211.

That is, the fourth wavelength conversion portion 238 is provided on the opposite surface of the first wavelength conversion portion 235 in the first rotating body 210, and the third wavelength conversion portion 238 is provided on the opposite surface of the second wavelength conversion portion 236 in the second rotating body 220. That is, the first wavelength conversion portion 235 and the fourth wavelength conversion portion 238 are arranged overlapping with each other in the first rotating body 210 as illustrated in Fig. 10 and Fig. 11, and the second wavelength conversion portion 236 and the third wavelength conversion portion 237 are arranged overlapping with each other in the second rotating body 220 as illustrated in Fig. 10.

From a control portion 234, the motor drive signals are supplied to the motors 231 and 232 respectively, and the driving current is supplied to the LDs 31A and 31B.

In the present embodiment, the light transmitted through the front side half mirror 91 is converged by a first converging lens 251 and radiated toward the first irradiation range (see a sign 251A indicated by a solid line in Fig. 11) of the front surface 212 of the first rotating body 210. The first converging lens 251 is provided facing the first wavelength conversion portion 235. Therefore, the blue fluorescence is generated from the first irradiation range 251A irradiated with the excitation light of the first wavelength conversion portion 235 illustrated in Fig. 11.

In contrast, the light reflected at the front side half mirror 91 illustrated in Fig. 10 is reflected at the first reflection mirror 71, converged at a second converging lens 252, and radiated toward the second irradiation range (not shown in the figure) of the front surface 222f of the second rotating body 220. The second converging lens 252 is provided facing the second wavelength conversion portion 236. Therefore, the green fluorescence is generated from the second irradiation range irradiated with the excitation light of the second wavelength conversion portion 236.

On the other hand, the light transmitted through the rear side half mirror 92 turns to a fourth converging lens 254, is converged at the lens 254, and is emitted toward the fourth irradiation range (see 254A indicated by a broken line in Fig. 11) of the rear surface 213 of the first rotating body 210. The fourth converging lens 254 is provided facing the fourth wavelength conversion portion 238. Therefore, the umber fluorescence is generated from the fourth irradiation range 254A irradiated with the excitation light of the fourth wavelength conversion portion 238 illustrated in Fig. 11.

In contrast, the light reflected at the rear side half mirror 92 illustrated in Fig. 10 is reflected at the second reflection mirror 72, converged at a third converging lens 253, and radiated toward the third irradiation range (not shown in the figure) of the rear surface 223 of the second rotating body 220. The third converging lens 253 is provided facing the third wavelength conversion portion 237. Therefore, the red fluorescence is generated from the third irradiation range irradiated with the excitation light of the third wavelength conversion portion 237.

Note that the first irradiation range 251A, the second irradiation range (not shown in the figure), the third irradiation range (not shown in the figure) and the fourth irradiation range 254A are circles of the same diameter, and are set larger than the width dimension w beforehand.

In the present embodiment, the first irradiation range 251A of the first converging lens 251 and the fourth irradiation range 254A of the fourth converging lens 254 are set in different areas across a line segment passing through the rotating shaft 32c as illustrated in Fig. 11.

Though illustrations are omitted, the second irradiation range of the second converging lens 252 and the third irradiation range of the third converging lens 253 are set in the different areas across the line segment passing through the rotating shaft 32c.

Specifically, in the present embodiment, as illustrated in Fig. 11, the first irradiation range 251A positioned on the front surface 212 of the first rotating body 210 and the fourth irradiation range 254A positioned on the rear surface 213 are position-shifted by 180 degrees across the rotating shaft 32c. In addition, though illustrations are omitted, the second irradiation range positioned on the front surface 222 of the second rotating body 220 and the third irradiation range positioned on the rear surface 223 are position-shifted by 180 degrees across the rotating shaft 32c.

As a result, when the excitation light is radiated toward the wavelength conversion portions 235 and 238 provided in the first rotating body 210 and the wavelength conversion portions 236 and 237 provided in the second rotating body 220, the excitation light is prevented from being simultaneously radiated toward the almost same point on the front surface 212f and the rear surface 213 of the first rotating body 210, and from being simultaneously radiated toward the almost same point on the front surface 222 and the rear surface 3223 of the second rotating body 220. As a result, the occurrence of the defect can be surely prevented, the defect being that one part is irradiated with the excitation light simultaneously from two directions and the temperature of the phosphor suddenly rises, thereby causing the remarkable decline of the conversion efficiency.

In addition, by appropriately setting a separation distance of the first converging lens 251 and the second converging lens 252 and a separation distance L of the fourth converging lens 254 and the third converging lens 253, the mutual interference of a first fluorescence pickup lens 261 and a second fluorescence pickup lens 262 arranged on the side of the front surfaces 212 and 222 and the mutual interference of a third fluorescence pickup lens 263 and a fourth fluorescence pickup lens 264 arranged on the side of the rear surfaces 213 and 223 are surely prevented, and the miniaturization can be realized.

In addition, one of the wavelength conversion portions 235 and 238 is provided respectively on the front surface 212f and the rear surface 213 of the first rotating body 210, and one of the wavelength conversion portions 236 and 237 is provided respectively on the front surface 222 and the rear surface 223 of the second rotating body 220. As a result, the respective rotating bodies 210 and 220 can be miniaturized and made light in weight. Therefore, the motors 331 and 332 are miniaturized compared to the motors 33 and 133.

Then, the light source device 3C is configured by adjacently and parallelly arranging the rotating bodies 210 and 220 that are miniaturized and made light weight. As a result, though the number of components is slightly increased from the above-described embodiments, the number of components is reduced compared to the conventional configuration, individual structural members are miniaturized and made light weight, and the weight reduction and miniaturization of the entire light source device 3C can be realized.

Note that the other actions and effects are similar to those of the above-described embodiments.

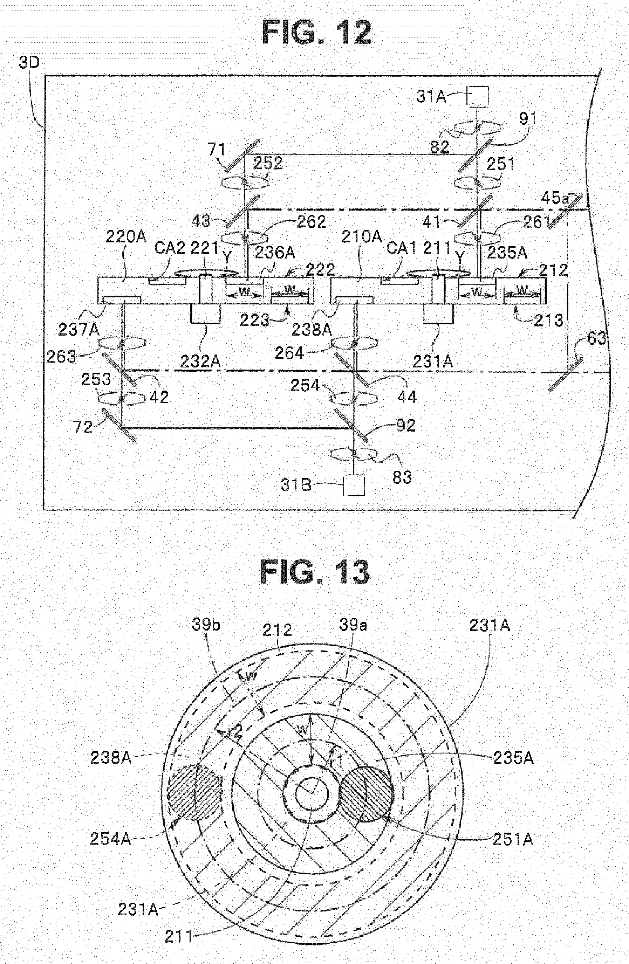

A modification of the light source device including two rotating bodies will be described with reference to Fig. 12 and Fig. 13.

A light source device 3D of the present embodiment includes two rotating bodies 210A and 220A instead of the two rotating bodies 210 and 220, and is provided with a first motor 231A that rotationally drives a first rotating body 210A and a second motor 232A that rotationally drives a second rotating body 220A. The other components are similar to those of the embodiments described above, the same signs are attached to the same members, and the descriptions are omitted.

The first rotating body 210A and the second rotating body 220A are in the almost similar configuration, and are planar disks. On the center positions of the respective rotating bodies 210A and 220A, rotating shafts 211A and 221A are integrally provided. The respective rotating shafts 211A and 221A are provided with the motors 231A and 232A respectively.

A first wavelength conversion portion 235A is provided on the front surface 212 of the first rotating body 210A as illustrated in Fig. 12 and Fig. 13, and a second wavelength conversion portion 236A is provided on the front surface 222 of the second rotating body 220A as illustrated in Fig. 12. Then, a third wavelength conversion portion 237A is provided on a rear surface 223 of the second rotating body 220A, and a fourth wavelength conversion portion 238A is provided on the rear surface 213 of the first rotating body 210A.

As illustrated in Fig. 13, in the first rotating body 210A, the first wavelength conversion portion 235A is configured including the first phosphor in the first annular area A1 formed along the first circle 39a formed on the front surface 212 with the radius r1 from the center of the rotating shaft 211. The width of the first annular area CA1 is w for example.

The fourth wavelength conversion portion 238A is configured including the fourth phosphor in the fourth annular area CA4 of the width w formed along the second circle 39b formed on the rear surface 213 with the radius r2 from the center of the rotating shaft 211.

Then, in the front view of the first rotating body 210A from the front surface 212 side, the first wavelength conversion portion 235A indicated by a solid line in Fig. 13 and the fourth wavelength conversion portion 238A indicated by a broken line are separated without overlapping, and concentrically arrayed with the rotating shaft 211 as the center.

Note that, though illustrations are omitted, in the second rotating body 220A, the second wavelength conversion portion 236A is configured including the third phosphor in the second annular area CA2 formed along the first circle 39a formed on the front surface 222 with the radius r1 from the center of the rotating shaft 221. The width of the second annular area CA2 is w for example.

The third wavelength conversion portion 237A is configured including the second phosphor in the third annular area CA3 of the width w formed along the second circle 39b formed on the rear surface 223 with the radius r2 from the center of the rotating shaft 221.

Then, in the front view of the second rotating body 220A from the front surface 212 side, the second wavelength conversion portion 236A provided on the front surface 212 and the third wavelength conversion portion 237A provided on the rear surface 32r are separated without overlapping, and concentrically arrayed with the rotating shaft 32c as the center.

Therefore, a first irradiation range 251B of the first converging lens 251 provided on the front surface 212 side of the first rotating body 210A and a fourth irradiation range 254B of the fourth converging lens 254 provided on the rear surface 213 are separated without overlapping in the view from one surface side as illustrated in Fig. 13.

Note that, though illustrations are omitted, the second irradiation range of the second converging lens 252 provided on the front surface 222 side of the second rotating body 220A and the third irradiation range of the third converging lens 253 provided on the rear surface 223 side are separated without overlapping in the view from one surface side.

According to the present embodiment, outer diameters of the rotating bodies 210A and 220A become larger than outer diameters of the rotating bodies 210 and 220; however, the first irradiation range 251B and the fourth irradiation range 254B are provided separately in the view from one surface side in the first rotating body 210A, and the second irradiation range and the third irradiation range of the second rotating body 220A are provided separately in the view from one surface side.

As a result, when the excitation light is radiated toward the wavelength conversion portions 235A, 236A, 237A and 238A provided in the individual rotating bodies 210A and 220A, the defect that the excitation light is simultaneously radiated toward the almost same point on the front surfaces 212 and 222 and the rear surfaces 3213 and 223r of the individual rotating bodies 210A and 220A can be dissolved.

In addition, the radii of the third wavelength conversion portion 237A and the fourth wavelength conversion portion 238A provided on the outer peripheral side of the rear surfaces 213 and 223 of the respective rotating bodies 210A and 220A are larger than the radii of the first wavelength conversion portion 235A and the second wavelength conversion portion 236A provided on the side of the rotating shafts 211 and 221 side. Therefore, by providing the third phosphor having the characteristic that conversion efficiency of the wavelength easily declines due to the rise of the temperature in the third wavelength conversion portion 237A and providing the fourth phosphor in the fourth wavelength conversion portion 238A, the decline of the conversion efficiency due to the rise of the temperature of the phosphor can be prevented.

The other actions and effects are similar to those of the above-described embodiments.

The present invention is not limited to the above-described embodiments and modifications and can be variously changed and modified or the like without changing a subject matter of the present invention.

The present application is filed with Japanese Patent Application No. 2014-212803 filed in Japan on October 17, 2014 as a base of a claim of priority, and the above-described disclosure content is cited in the present description, scope of claims and drawings.

## Claims

1. A light source device comprising:
a rotating body configured to be rotated with a rotating shaft as a center;
a plurality of phosphors arranged on a plurality of circumferences of different radii with the rotating shaft as the center in the rotating body, and configured to be excited by being irradiated with light and generate fluorescence; and
a light source portion configured to radiate excitation light for making one phosphor arranged on a predetermined circumference among the plurality of phosphors generate the fluorescence, and configured to radiate the excitation light to an area other than a straight line passing from the rotating shaft through an irradiation position at which the one phosphor is irradiated with the excitation light, in a phosphor different from the one phosphor among the plurality of phosphors.

2. The light source device according to claim 1,
wherein the plurality of phosphors are provided in annular portions with n pieces (n: 2 or larger) of the circumferences of the different radii as center lines with the rotating shaft as the center,
the light source portion radiates the light for making the respective phosphors arranged in the n pieces of annular portions generate the fluorescence, and the light for making the fluorescence be generated is radiated to the phosphors in the annular portions different in each of n pieces of areas equally divided into n with the rotating shaft as the center in the rotating body among the plurality of phosphors.

3. The light source device according to claim 1, comprising
a multiplexing portion including:
a dichroic mirror provided on an optical path of the excitation light that makes the one phosphor generate the fluorescence, and configured to transmit the excitation light and reflect the fluorescence generated by the one phosphor irradiated with the excitation light; and
a dichroic mirror different from the dichroic mirror, provided on an optical path of the excitation light that makes a phosphor different from the one phosphor generate the fluorescence, and configured to transmit the excitation light, reflect the fluorescence generated by the phosphor different from the one phosphor irradiated with the excitation light, and multiplex the fluorescence generated by the phosphor different from the one phosphor and the fluorescence reflected by the dichroic mirror.

4. The light source device according to claim 1,
wherein the plurality of phosphors generate the fluorescence of respectively different wavelengths, and
the phosphor with greatest degradation of generation efficiency of the fluorescence due to a temperature among the plurality of phosphors is arranged in an annular portion provided on an outmost peripheral side of the rotating body among the annular portion in plurality of different radii from the rotating shaft.

5. A light source device comprising:
a rotating body configured to be rotated with a rotating shaft as a center and including a front surface and a rear surface;
a control portion configured to control a drive portion that rotationally drives the rotating shaft in order to rotate the rotating body around the shaft;
a front surface side wavelength conversion portion provided on a circumference with the rotating shaft as the center on the front surface of the rotating body, and configured to receive light and generate light of a wavelength different from a wavelength of the light;
a rear surface side wavelength conversion portion provided on a circumference with the rotating shaft as the center on the rear surface of the rotating body, and configured to receive light and generate light of a wavelength different from a wavelength of the light;
an irradiation portion configured to irradiate a predetermined position of the front surface side wavelength conversion portion provided on the front surface of the rotating body with light, and further irradiate an area different from the predetermined position of the rear surface side wavelength conversion portion provided on the rear surface of the rotating body with light; and
a multiplexing portion configured to multiplex the light generated from the front surface side wavelength conversion portion and the light generated from the rear surface side wavelength conversion portion.

6. The light source device according to claim 5, comprising
one rotating body,
wherein the rotating body includes
a first front surface side wavelength conversion portion provided on a center side of the front surface, and a second front surface side wavelength conversion portion provided on an outer peripheral side of the front surface and separated from the first front surface side wavelength conversion portion, and includes
a first rear surface side wavelength conversion portion provided on a center side of the rear surface, and a second rear surface side wavelength conversion portion provided on an outer peripheral side of the rear surface and separated from the first rear surface side wavelength conversion portion,
the first front surface side wavelength conversion portion and the first rear surface side wavelength conversion portion are provided on a circumference separated by a first distance with the rotating shaft as the center, and the second front surface side wavelength conversion portion and the second rear surface side wavelength conversion portion are provided on a circumference separated by a second distance with the rotating shaft as the center.

7. The light source device according to claim 6,
wherein the irradiation portion includes:
a first front surface side irradiation portion provided facing the first front surface side wavelength conversion portion and configured to radiate the light toward a first irradiation range of the front surface;
a second front surface side irradiation portion provided facing the second front surface side wavelength conversion portion and configured to radiate the light toward a second irradiation range of the front surface;
a first rear surface side irradiation portion provided facing the first rear surface side wavelength conversion portion and configured to radiate the light toward a third irradiation range of the rear surface, which is a position different from an opposite surface side of the first irradiation range irradiated with the light by the first front surface side irradiation portion; and
a second rear surface side irradiation portion provided facing the second front surface side wavelength conversion portion and configured to radiate the light toward a fourth irradiation range of the rear surface, which is a position different from an opposite surface side of the second irradiation range irradiated with the light by the second front surface side irradiation portion.

8. The light source device according to claim 5, comprising
one rotating body,
wherein the rotating body includes:
a first wavelength conversion portion provided on the circumference formed on the front surface or the rear surface of the rotating body, separated by a first distance with the rotating shaft as the center;
a second wavelength conversion portion provided on the circumference of the surface provided with the first wavelength conversion portion, separated by a third distance with the rotating shaft as the center;
a third wavelength conversion portion provided on the circumference of the surface different from the surface provided with the first wavelength conversion portion, separated by a second distance with the rotating shaft as the center; and
a fourth wavelength conversion portion provided on the circumference of the surface provided with the second wavelength conversion portion, separated by a fourth distance with the rotating shaft as the center, and
in a state of having a front view of the front surface or the rear surface, the first wavelength conversion portion, the second wavelength conversion portion, the third wavelength conversion portion and the fourth wavelength conversion portion are arrayed on concentric circles of the rotating shaft without overlapping.

9. The light source device according to claim 8,
wherein the irradiation portion includes a first irradiation portion facing a predetermined position of the first wavelength conversion portion and a second irradiation portion facing a predetermined position of the second wavelength conversion portion, which are provided on one surface side of the rotating body, and a third irradiation portion facing a predetermined position of the third wavelength conversion portion and a fourth irradiation portion facing a predetermined position of the fourth wavelength conversion portion, which are provided on an opposite surface side of the rotating body, and
the four irradiation portions are provided in twos on one surface side and on the opposite surface side of the rotating body such that pickup lenses that converge the light of different wavelengths generated from each of the wavelength conversion portions do not mutually interfere.

10. The light source device according to claim 5, comprising:
two rotating bodies;
wherein each of the rotating bodies includes:
a front surface side wavelength conversion portion provided on the circumference of the front surface of the rotating body, separated by a first distance with the rotating shaft as the center; and
a rear surface side wavelength conversion portion provided on the circumference formed on the rear surface of the rotating body, separated by the first distance with the rotating shaft as the center.

11. The light source device according to claim 5, comprising:
two rotating bodies;
wherein each of the rotating bodies includes:
a wavelength conversion portion on one surface side provided on the circumference of the front surface or the rear surface of the rotating body, separated by a first distance with the rotating shaft as the center; and
a wavelength conversion portion on an opposite surface side provided on the circumference formed on the surface different from the surface provided with the wavelength conversion portion, separated by a second distance with the rotating shaft as the center.

12. The light source device according to any one of claim 5 to claim 11,
wherein the light emitted from the light source portion is excitation light, and the wavelength conversion portions are two or more kinds of phosphors that receive the excitation light and emit predetermined fluorescence.

13. The light source device according to any one of claim 6, claim 8, claim 11 and claim 12,
wherein a phosphor having a characteristic that reduction of generation efficiency of the light accompanying rise of a temperature is great among the phosphors is arranged on an outer peripheral side with respect to a rotating shaft side of the rotating body.

14. A light source device comprising:
a rotating body configured to be rotated with a rotating shaft as a center and including a front surface and a rear surface;
a control portion configured to control a drive portion that rotationally drives the rotating shaft in order to rotate the rotating body around the shaft;
a front surface side wavelength conversion portion provided on a circumference with the rotating shaft as the center on the front surface of the rotating body, and configured to receive light from an irradiation portion that radiates light and generate light of a wavelength different from a wavelength of the light;
a rear surface side wavelength conversion portion provided on a circumference different from the front surface side wavelength conversion portion with the rotating shaft as the center on the rear surface of the rotating body, and configured to receive the light from the irradiation portion and generate light of a wavelength different from a wavelength of the light; and
a multiplexing portion configured to multiplex the light generated from the front surface side wavelength conversion portion and the light generated from the rear surface side wavelength conversion portion.
